# FASCICULE DE BREVET EUROPEEN

(11) **EP 3 386 454 B1**
(45) Date de publication et mention de la délivrance du brevet: **15.04.2020**
(21) Numéro de dépôt: 16809056.1
(22) Date de dépôt: 09.12.2016
(51) Int. Cl.: A61F 13/00, A61F 13/02, A61K 9/70, A61F 13/40

(54) **PANSEMENT POUR SOINS DE LA PEAU EN MILIEU HUMIDE**
VERBAND ZUR HAUTPFLEGE IN FEUCHTER UMGEBUNG
DRESSING FOR SKIN CARE IN A MOIST ENVIRONMENT

(30) Priorité: 09.12.2015 FR 1562074
(43) Date de publication de la demande: 17.10.2018
(73) Titulaire: Droche, Emile, 92250 La Garenne Colombes (FR)
(72) Inventeur: Droche, Emile, 92250 La Garenne Colombes (FR)
(74) Mandataire: V.O.
(86) Numéro de dépôt international: PCT/EP2016/080468
(87) Numéro de publication internationale: WO 2017/097993

(56) Documents cités:
- WO-A2-2013/039713
- CA-A1- 2 586 948
- US-A- 3 367 332
- US-A1- 2014 276 475

## Description

La présente invention concerne un système de pansement prévu pour s'appliquer sur la peau d'un patient en maintenant un milieu humide, ainsi qu'un procédé de mise en oeuvre de ce pansement.

Le pansement courant utilisé depuis longtemps comporte un produit formant une compresse, qui est recouvert par un film ou une membrane dont les contours sont adhésifs pour plaquer cette compresse sur la peau.

La fonction première de ce type de pansement est de protéger une plaie superficielle sur la peau d'un patient, en la recouvrant pour la maintenir au sec et à l'abri du milieu extérieur pouvant l'affecter. Cette protection à renouveler plusieurs fois par jour, est maintenue pendant un temps nécessaire pour obtenir une cicatrisation suffisante de la peau.

Ce type de pansement qui est économique, est toujours en cours d'utilisation. Il peut comporter une fonction antiseptique légère pour éviter une infection de la plaie, permettant alors d'espacer les renouvèlements, qui sont fait par exemple une fois par jour.

La plaie peut être causée par toutes sortes de problèmes, comprenant notamment une coupure, une brûlure, un bouton, un ulcère ou une maladie de la peau.

Un problème qui se pose avec ce type de pansement est que l'on a habituellement sur la compresse des adhésions du tissu de cicatrisation en train de se former sur la plaie, qui est alors arraché lors du retrait suivant. On ralentit ainsi le cycle de cicatrisation, et retarde la guérison. En particulier la guérison des ulcères peut devenir impossible.

Pour limiter une adhésion de la compresse il est connu d'utiliser en face de la plaie une grille en polymère et / ou un produit du type hydro-colloïde, qui peut être associé avec de la vaseline. Toutefois cette solution ne donne pas toujours entièrement satisfaction.

Un type de pansement haut-de-gamme proposé aujourd'hui, qui est plus onéreux, favorise la cicatrisation en maintenant un milieu humide grâce à une masse hydro-colloïde.

De plus certains de ces pansements peuvent absorber des saignements. Dans ce cas ce type de pansement doit être remplacé plusieurs fois au cours du traitement, généralement au bout de 24 heures la première fois, et ensuite pour chaque période de 48 heures.

Une thérapie particulière réalise une pression négative dans le pansement maintenant un milieu humide et antiseptique, afin de favoriser notamment l'absorption de produits par la peau. Il est connu d'utiliser différents types de pompe pour créer une pression négative, toutefois ces systèmes présentent des coûts élevés, et nécessitent une énergie pour l'actionnement de la pompe.

En variante un type de pansement maintenant une pression négative dans un milieu humide, présenté notamment par le document US-A1-20050070835, comporte un réservoir plat prévu pour être maintenu sur le pansement, contenant des moyens chimiques ou électrochimiques qui absorbent l'oxygène afin de réduire la pression de gaz dans ce réservoir.

Toutefois ce type de moyen prévu pour absorber l'oxygène est complexe à mettre en œuvre, et augmente les coûts. Un pansement prévu pour contenir un fluide de traitement est décrit dans le document US 3,367,332. Ce pansement comporte une membrane, une zone périphérique adhésive et un tube permettant d'injecter le fluide sous la zone centrale de la membrane.

La présente invention a notamment pour but d'éviter ces inconvénients de la technique antérieure.

Elle propose à cet effet pour un premier aspect un pansement pour soins de la peau, comportant une compresse principale recouverte par une membrane présentant un contour adhésif dépassant de cette compresse, prévu pour être appliqué sur la peau, ce pansement comportant une ouverture d'entrée d'un liquide de traitement sur la compresse principale, la membrane présentant sur au moins une partie de la surface en regard de cette compresse une perméabilité permettant des échanges gazeux, ce pansement étant remarquable en ce que l'ouverture d'entrée comporte un bras de compresse qui part d'un côté de la compresse principale pour traverser le contour adhésif.

Un avantage de ce pansement est que d'une part l'ouverture d'entrée permet de contrôler un débit du liquide de traitement pour imbiber la compresse, et que d'autre part la perméabilité de la membrane au gaz permet pendant l'utilisation du pansement de laisser des gaz s'échapper afin d'éviter
une pression dans ce pansement. De plus l'échange gazeux permet d'éviter la macération de la plaie et permet le renouvellement du liquide.

Le pansement selon l'invention peut comporter de plus une ou plusieurs des caractéristiques suivantes, qui peuvent être combinées entre elles.

Selon un mode de réalisation du premier aspect, le pansement comporte en plus de la perméabilité, une ouverture de sortie pour l'air contenu dans la compresse principale.

Selon un autre mode de réalisation du premier aspect, l'ouverture de sortie peut comporter un bras de compresse qui part d'un côté de la compresse principale pour traverser le contour adhésif. Ce type d'entrée ou de sortie est simple et économique à réaliser.

Pour un second aspect, l'invention propose un pansement pour soins de la peau, comportant une compresse principale recouverte par une membrane présentant un contour adhésif dépassant de cette compresse, prévu pour être appliqué sur la peau, ce pansement comportant une ouverture d'entrée d'un liquide de traitement sur la compresse principale, la membrane présentant sur au moins une partie de la surface en regard de cette compresse une perméabilité permettant des échanges gazeux, ce pansement étant remarquable en ce que l'ouverture d'entrée forme un perçage de la membrane, disposé sur le contour adhésif à distance du rebord de cette membrane.

Un mode de réalisation du second aspect, comporte en plus de la perméabilité, une ouverture de sortie pour l'air contenu dans la compresse principale, l'ouverture de sortie formant un perçage de la membrane, disposé sur le contour adhésif à distance du rebord de cette membrane.

Selon un autre mode de réalisation du second aspect, l'ouverture de sortie peut former un perçage de la membrane, disposé sur le contour adhésif à distance du rebord de cette membrane.

Dans ce cas, le perçage de l'ouverture d'entrée ou de sortie comporte avantageusement en dessous de la membrane un morceau de compresse qui est séparé de la compresse principale. La séparation réalise un certain freinage du débit, ce qui impose une pression d'alimentation pour obtenir l'écoulement du liquide.

Selon encore un autre mode de réalisation du second aspect, la membrane comporte un perçage d'entrée, une rondelle, par exemple rigide, est disposée sous cette membrane autour de ce perçage pour réaliser un centrage d'un embout de versement du liquide. On réalise ainsi de manière simple un moyen tactile pour trouver le perçage, et une aide au positionnement de l'embout.

Selon un autre mode de réalisation du premier et du second aspect, le pansement comporte un moyen de fermeture de l'ouverture d'entrée, comprenant une partie souple venant sur cette ouverture. On protège ainsi l'entrée de l'arrivée de salissures.

Selon encore un autre mode de réalisation du premier et du second aspect, la perméabilité permettant des échanges gazeux est formée par une microstructure ou des micro-perforations de la membrane.

Selon un mode de réalisation, le pansement comporte un réservoir intégré prévu pour recevoir le liquide. Dans ce cas on n'a pas besoin d'utiliser un moyen additionnel pour réaliser le remplissage du liquide.Selon un mode suivant de réalisation du second aspect, le réservoir intégré peut être formé par une feuille étanche supérieure fixée par son contour sur la membrane, ou sur une feuille étanche inférieure qui est elle-même fixée sur la membrane.

Selon un autre mode suivant de réalisation du second aspect, le pansement avec réservoir intégré comporte une vanne d'ouverture du réservoir vers la compresse principale, commandée par un bouton. Le bouton permet une commande simple et facile de l'ouverture du réservoir pour réaliser l'infiltration de la compresse.

Selon encore un autre mode suivant de réalisation du second aspect pour un pansement avec réservoir intégré, la membrane ne comporte pas d'ouverture de sortie en plus de la perméabilité. Le réservoir intégré permettant un débit d'imprégnation de la compresse lent par capillarité, la perméabilité aux échanges gazeux de cette compresse est suffisante pour vider l'air contenu dedans.

L'invention sera mieux comprise et d'autres caractéristiques et avantages apparaîtront plus clairement à la lecture de la description ci-après donnée à titre d'exemple, en référence aux dessins annexés dans lesquels :
- la figure 1 est une vue de dessus d'un pansement selon l'invention ;
- la figure 2 est une vue de dessous de ce pansement ;
- la figure 3 est une vue en coupe suivant le plan de coupe III-III d'un flacon remplissant de liquide ce pansement ;
- la figure 4 est une vue extérieure de ce flacon remplissant le pansement ;
- la figure 5 est une vue de dessus d'un pansement suivant une première variante ;
- la figure 6 est une vue de dessous de ce pansement ;
- les figures 7 et 8 sont des vues de détail respectivement de l'entrée et de la sortie de ce pansement ;
- la figure 9 est une vue extérieure d'un flacon remplissant ce pansement ;
- la figure 10 est une vue en coupe suivant le plan de coupe X-X d'un flacon remplissant de liquide ce pansement ;
- la figure 11 est une vue en coupe axiale suivant le plan de coupe X-X d'un flacon remplissant de liquide un pansement similaire comportant une rondelle de guidage ;
- les figure 12 et 13 sont des vues respectivement de détail et d'ensemble d'un pansement similaire comportant une languette de fermeture du perçage d'entrée présentée ouverte puis fermée ;
- Les figures 14 et 15 présentent un pansement comprenant un réservoir intégré de liquide, vu respectivement de dessus, et en coupe suivant le plan de coupe XV-XV ;
- la figure 16 présente ce pansement vu de dessous ;
- les figures 17 et 18 sont des vues en coupe suivant le plan de coupe XV-XV, de ce réservoir avec une vanne d'ouverture présentée respectivement en position fermée et ouverte ; et
- la figure 19 est une vue suivant le même plan de coupe d'un pansement présentant un réservoir suivant une variante.

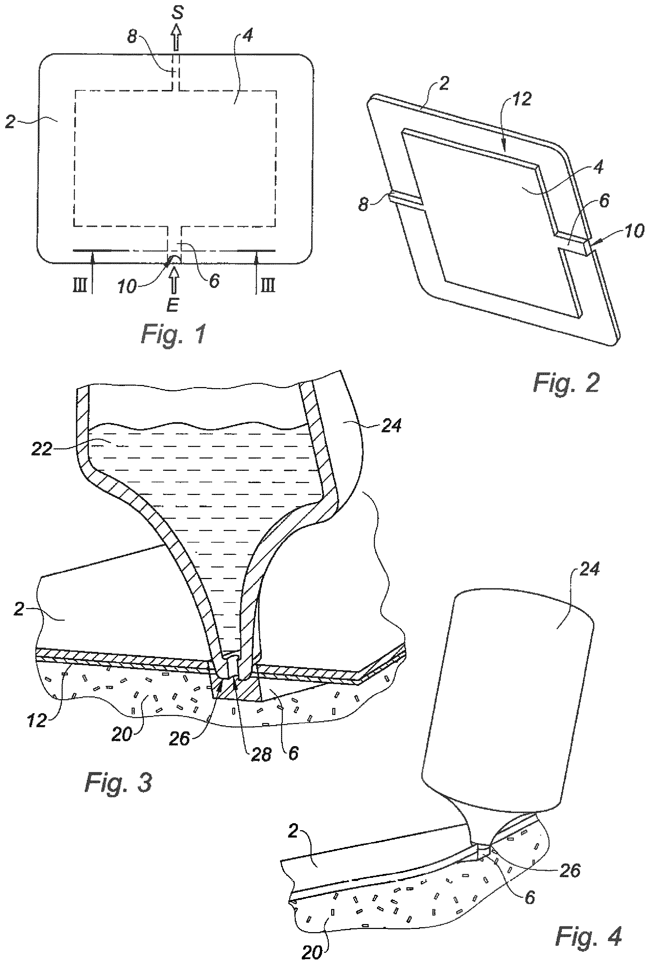

Les figures 1 et 2 présentent un pansement de forme rectangulaire comportant une membrane extérieure souple 2 recevant en dessous une compresse plate 4, qui est entourée par une bordure de la membrane de largeur constante recevant un revêtement adhésif autocollant 12.

La membrane 2 présente au moins sur la surface en regard de la compresse 4 une perméabilité permettant des échanges gazeux, grâce par exemple à une microstructure ou à des micro-perforations.

Avantageusement la membrane 2 est réalisée en matière plastique, en particulier on peut utiliser un polyuréthane qui est souple et économique.

Sur un côté du rectangle la compresse 4 se prolonge par un bras d'entrée 6 venant jusqu'au contour extérieur de la membrane 2, se terminant par un arc de cercle concave 10 qui est découpé aussi sur cette membrane. Sur le côté opposé, la compresse 4 se prolonge par un bras de sortie 8 présentant une largeur réduite.

Une fois appliqué sur la peau, le revêtement adhésif 12 de la membrane 2 ferme entièrement le contour de la compresse 4, hormis les extrémités des bras d'entrée 6 et de sortie 8 qui restent accessibles par l'extérieur.

Les figures 3 et 4 présentent un flacon 24 contenant un liquide de traitement 22, comportant un embout effilé 26 se terminant par un arrondi présentant un perçage axial 28.

Une fois le pansement appliqué sur la peau, l'opérateur ajuste l'embout 26 du flacon 24 sur la découpe en arc de cercle concave 10 du bras d'entrée de la compresse 6 et de la membrane 2, et injecte le liquide 22 avec une légère pression, par exemple en pressant sur le flacon qui est en plastique souple, pour le propulser par capillarité dans le bras d'entrée 6 puis dans la compresse 4 entière.

Pendant cette injection l'air contenu dans le volume fermé formé sous la membrane 2, rempli par la compresse 4, est chassé à l'opposé par le bras de sortie 8 qui est perméable à cet air.

En particulier en prévoyant une membrane 2 transparente ou translucide, on peut suivre l'avancée du liquide au travers de la compresse 4, et vérifier le remplissage complet. De plus on peut prévoir un liquide coloré qui permet de suivre plus facilement son avancement.

On peut aussi s'assurer du remplissage du pansement par un écoulement du liquide par le bras de sortie 8, qui traduirait un remplissage complet. Les orifices d'entrée 6 et de sortie 8 permettent de plus en cours d'utilisation du pansement, un écoulement de liquide évitant une montée en pression dedans lors d'une compression occasionnelle de ce pansement, par exemple dans le cas où il se trouve sur une articulation d'un membre qui est plié.

En complément quelque temps après le remplissage initial du pansement, on peut en procédant de la même manière effectuer une réalimentation avec le même liquide, ou un liquide différent au cours de l'évolution du traitement, pour compenser une évaporation ou des légers suintements de liquide.

La largeur réduite du bras de sortie 8 par rapport à celle du bras d'entrée 6 est adaptée pour permettre un écoulement d'air qui se fait facilement par une petite section, alors que la section d'entrée pour le liquide est plus importante.

En plus d'une sortie de gaz par le bras de sortie 8, une production gazeuse dans le pansement, par exemple d'oxygène, est évacuée de manière simple sur toute la surface de cette compresse par la perméabilité gazeuse de la membrane 2.

La section réduite des orifices d'entrée et de sortie aux extrémités des bras 6, 8 limite la possibilité d'entrée de bactéries dans le pansement. De plus ces orifices d'entrée et de sortie sont éloignées de la compresse principale 4 par la longueur des bras 6, 8, ce qui limite la possibilité de migration des bactéries vers cette compresse.

On obtient une compresse imprégnée par un liquide qui est confiné sur la plaie, comportant au moins au départ un antiseptique. La composition du liquide peut évoluer pendant le traitement. Le pansement peut être gardé beaucoup plus longtemps grâce à la possibilité de rechargement en liquide.

En particulier on peut réaliser avec le même pansement des traitements cutanés ou transcutanés comportant une évolution des produits médicaux utilisés. Ce type de traitement est particulièrement intéressant pour soigner des lésions comme des aphtes, des boutons, des ulcères, des allergies, ou pour réduire des cicatrices.

Lors du retrait du pansement, le maintien de toute la surface de la plaie dans un milieu humide limite fortement le collage et l'arrachement des tissus régénérés. De plus le renouvellement du fluide permet de maintenir plus longtemps le pansement qui est changé moins souvent, ce qui réduit aussi le risque d'arrachement de ces tissus.

On peut utiliser tout type de réservoir pour le liquide d'apport, comprenant notamment des flacons en plastique ou en verre, des ampoules ou des seringues.

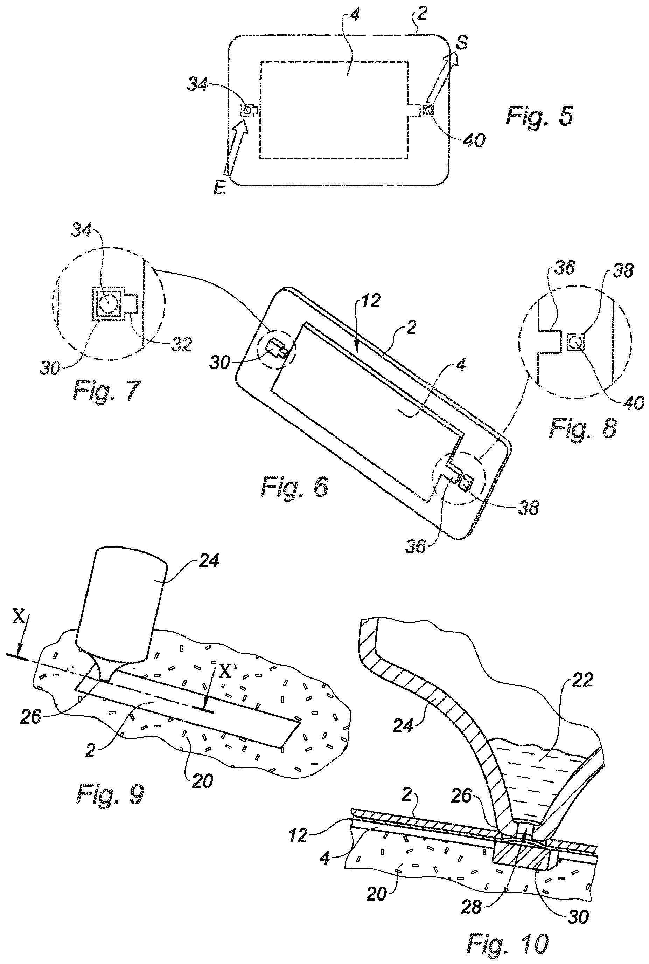

Les figures 5, 6, 7 et 8 présentent un pansement formant un rectangle allongé, comportant une compresse similaire 4 entourée par une bordure recevant le revêtement adhésif 12.

Sur un petit côté du rectangle, la bordure reçoit en dessous un système d'entrée éloigné du contour de la membrane 2, comprenant un morceau carré de compresse d'entrée 30 comportant sur un côté tourné vers la compresse principale 4 une pointe 32 séparée de cette compresse par une petite distance. La membrane 2 comporte un perçage d'entrée 34 disposé au milieu du morceau carré d'entrée 30.

Sur le petit côté opposé du rectangle, la bordure reçoit en dessous un système de sortie comprenant un bras 36 relié à la compresse 4, et à une petite distance de son extrémité, un morceau carré de compresse de sortie 38 qui est éloigné du contour de la membrane 2. La membrane 2 comporte un perçage de sortie 40 disposé au milieu du morceau carré de compresse de sortie 38.

On obtient de cette manière après application du revêtement adhésif 12 sur la peau, des morceaux de compresse d'entrée 30 et de sortie 38 qui sont éloignés des bords de la membrane 2 pour donner une étanchéité de ces morceaux par rapport à l'extérieur.

On obtient aussi par la petite distance sans compresse sur l'entrée et la sortie, un passage freiné entre la compresse principale 4 et cette entrée ou sortie, qui permet un passage du liquide seulement dans le cas où une certaine pression est appliquée. Par simple capillarité on n'a pas de passage de liquide.

Les figures 9 et 10 présentent l'application de l'embout effilé 26 du flacon 24 sur le perçage d'entrée 34, ce qui permet en appliquant une pression sur le liquide 22, par exemple en pressant le flacon qui est souple, d'obtenir une imprégnation du morceau de compresse d'entrée 30, qui se communique par la pointe 32 et par la petite distance le séparant de la compresse principale 4, vers cette compresse pour l'imbiber entièrement.

On notera que le perçage d'entrée 34 recevant l'extrémité de l'embout effilé 26 qui se loge dedans, on obtient un centrage facile de cette embout qui vient directement en contact avec le morceau de compresse d'entrée 30, ce qui limite les pertes de liquide vers l'extérieur.

D'une manière similaire l'air contenu dans la compresse 4 est refoulé vers le bras de sortie 36, puis en passant par la petite distance, vers le morceau de compresse de sortie 38 communiquant avec le perçage de sortie 40.

Les petites distances sans compresse sur l'entrée et la sortie permettent également de recevoir le liquide de la compresse quand elle est saturée et comprimée par la flexion d'une articulation par exemple.

La pression d'entrée nécessaire grâce au freinage du passage du liquide sur l'entrée, permet de limiter les infiltrations éventuelles d'autres fluides vers la compresse 4, en particulier si les systèmes d'entrée et de sortie sont asséchés, ainsi que la migration des bactéries par la compresse, ce qui la protège de salissures ou d'infections.

De plus les morceaux de compresse d'entrée 30 et de sortie 38 étant éloignés des bords de la membrane 2, on a une continuité du collage de cette membrane sur tout son pourtour, ce qui donne une absence de risque de propagation du décollement à partir d'un passage d'entrée ou de sortie qui viendrait jusqu'à ce bord.

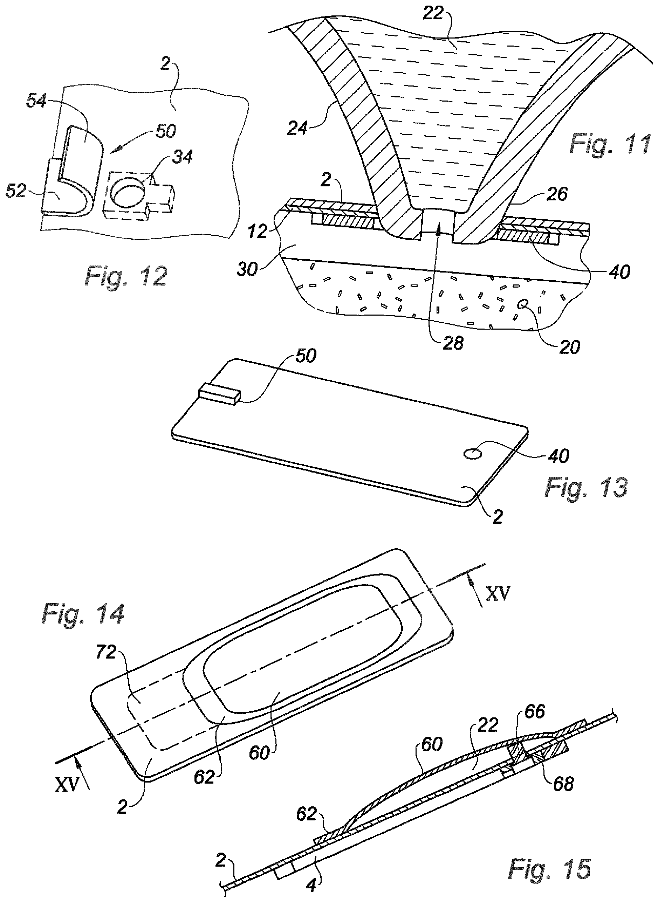

La figure 11 présente un pansement similaire à celui présenté précédemment, comportant de plus une rondelle, par exemple rigide, de guidage 40 disposée autour du perçage d'entrée 34, entre la membrane 2 et la compresse 4, qui est collée sous cette membrane par le revêtement adhésif 12.

La rondelle de guidage 40 présentant un perçage central axé sur le perçage d'entrée 34, constitue un moyen de centrage de l'embout effilé 26 du flacon 24 permettant de positionner et de maintenir cette extrémité sur une partie rigide, pour effectuer un transfert du liquide 22 en évitant un décalage du flacon qui causerait une perte de ce liquide.

De plus la rondelle 40 de guidage facilite pour une personne malvoyante la recherche du positionnement du perçage 34, en percevant cette pièce par un touché au travers de la membrane 2.

Les figures 12 et 13 présentent un pansement similaire comportant de plus une languette de fermeture 50 présentant une extrémité 52 collée sur la membrane 2 à proximité du perçage d'entrée 34, l'autre extrémité formant une partie souple 54 venant s'appliquer sur ce perçage d'entrée pour le maintenir fermé.

De cette manière on maintient en permanence le perçage d'entrée 34 fermé pour le protéger de salissures, et par un simple soulèvement de la partie souple 54 on découvre ce perçage pour une alimentation du pansement en liquide.

Les figures 14, 15 et 16 présentent un pansement comprenant au-dessus de la membrane 2 un réservoir intégré formé par une feuille étanche souple en matière plastique 60 fixée sur cette membrane par son contour 62. En particulier on peut fixer la feuille 60 sur la membrane 2 par un collage, notamment avec une colle polyuréthane, ou par une soudure, notamment avec une soudure haute fréquence.

Une vanne d'ouverture du réservoir 66 est disposée sur l'axe médian principal du pansement, sur un côté du réservoir 60, afin de permettre un passage du liquide de ce réservoir vers la compresse 4.

Le contour 62 de la feuille du réservoir 60 laisse du côté opposé à la vanne 66, au-dessus de la compresse 4, une partie libre 72 de la membrane 2 qui est découverte afin de permettre par sa perméabilité gazeuse une sortie des gaz contenus dans cette compresse.

De cette manière on obtient lors de l'imprégnation de la compresse 4, un flux de liquide venant de la vanne 66 qui imbibe progressivement cette compresse par capillarité en progressant suivant l'axe principal du pansement, et en chassant devant lui l'air qui est refoulé vers le côté opposé où se trouve la partie libre de la membrane 72 permettant l'évacuation des gaz.

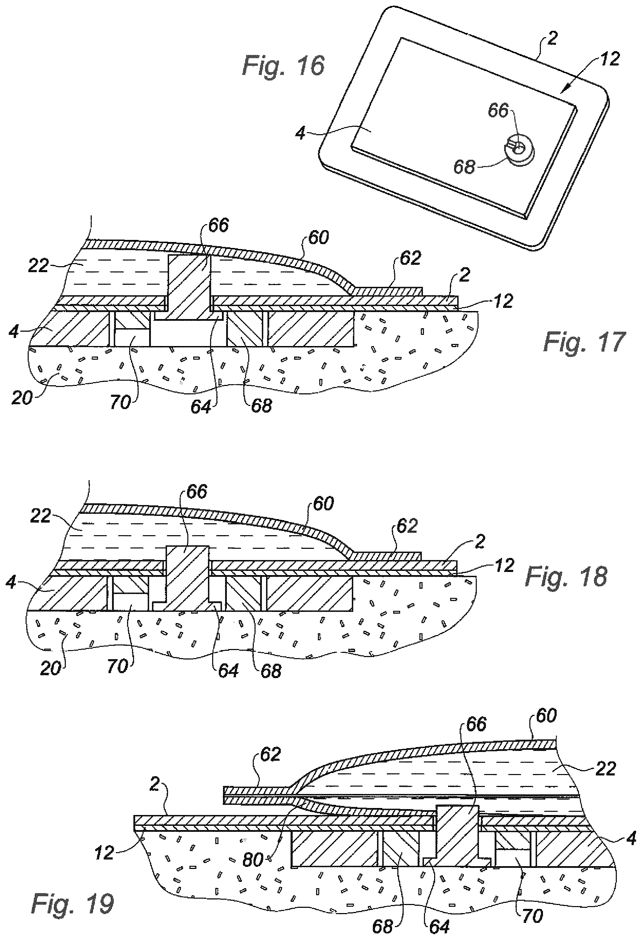

La figure 17 détaille la vanne 66 comprenant un bouton cylindrique traversant un perçage de la membrane 2, dont le sommet est ajusté sous la feuille souple 60 du réservoir rempli. La base du bouton cylindrique de la vanne 66 forme un épaulement circulaire 64 qui est au départ collé sous la membrane 2 par le revêtement adhésif 12, afin d'assurer une étanchéité fermant le réservoir.

L'épaulement 64 de la vanne 66 est entourée par une rondelle rigide 68 ajustée dans la compresse 4 et présentant au moins la même épaisseur, comprenant vers le bas un canal horizontal 70 traversant cette rondelle.

Après avoir mis en place le pansement sur la peau du patient 20, un appui sur le bouton de la vanne 66 au travers de la feuille souple 60 décolle l'épaulement 64 de la membrane 2. La rondelle rigide 68 permet la descente de la vanne 66 grâce à l'espace vertical qu'elle préserve en dessous de cette vanne. On obtient un passage du liquide 22 vers la compresse 4 en passant par le canal 70.

Contrairement au flacon posé sur le pansement présenté ci-dessus, nécessitant un transfert du liquide par un opérateur qui ne peut pas passer trop de temps, le réservoir intégré sur le pansement permet d'effectuer l'imprégnation de la compresse 4 pendant un temps long, avec un écoulement progressif du liquide au travers de la vanne 66. L'air contenu dans la compresse 4 peut alors s'écouler progressivement au travers de la partie libre de la membrane 72, sans nécessité de perçage de sortie particulier.

On obtient un pansement autonome ne comportant aucune ouverture vers l'extérieur, l'entrée du liquide vers la compresse 4 étant intégrée à l'intérieur du réservoir, ce qui lui donne un haut niveau de protection contre les salissures et les infections.

Le traitement du patient est simplifié avec une seule opération d'application du pansement sur la peau, pouvant être faite de manière rapide par un personnel peu qualifié, ce qui réduit les coûts. Le pansement autonome avec réservoir intégré ne nécessite aucun équipement complémentaire, en prévoyant en particulier une livraison de pansements comportant un réservoir déjà rempli du liquide de traitement.

En variante on peut prévoir des pansements livrés avec un réservoir vide, le remplissage se faisant par un bouchon d'entrée, ou par injection avec une seringue au travers de la feuille souple 60.

La figure 19 présente un pansement similaire, comportant un réservoir formé par une feuille supérieure souple 60 qui est fixée sur son pourtour sur une feuille inférieure souple 80. Une partie centrale de la feuille inférieure 80 comporte un perçage ajusté sur la vanne 66, le contour de ce perçage étant collé sur la membrane 2 afin d'assurer le passage du liquide vers cette vanne avec une étanchéité autour.

En position fermée le sommet de la vanne 66 est ajusté sous la feuille supérieure 60 du réservoir, le fonctionnement de cette vanne étant identique.

D'une manière générale le pansement selon l'invention permet un traitement non chirurgical d'une variété importante de plaies ou de problèmes de l'épiderme, comprenant une cicatrisation rapide grâce à la présence d'un milieu humide recevant des produits de traitement, et au maintien de ce pansement pendant un temps assez long sans problème d'accrochage des tissus régénérés, ce qui évite de les arracher.

## Revendications

1. Pansement pour soins de la peau, comportant une compresse principale (4) recouverte par une membrane (2) présentant un contour adhésif (12) dépassant de cette compresse, prévu pour être appliqué sur la peau, et comportant une ouverture d'entrée (6) d'un liquide de traitement (22) sur la compresse principale (4), la membrane (2) présentant sur au moins une partie de la surface en regard de cette compresse une perméabilité permettant des échanges gazeux,
ie pansement étant **caractérisé en ce que** l'ouverture d'entrée comporte un bras de compresse (6) qui part d'un côté de la compresse principale (4) pour traverser le contour adhésif (12).

2. Pansement selon la revendication 1, **caractérisé en ce qu'**il comporte en plus de la perméabilité, une ouverture de sortie (8) pour l'air contenu dans la compresse principale (4).

3. Pansement selon la revendication 2, **caractérisé en ce que** l'ouverture de sortie (8) comporte un bras de compresse qui part d'un côté de la compresse principale (4) pour traverser le contour adhésif (12).

4. Pansement pour soins de la peau, comportant une compresse principale (4) recouverte par une membrane (2) présentant un contour adhésif (12) dépassant de cette compresse, prévu pour être appliqué sur la peau, et comportant une ouverture d'entrée (34) d'un liquide de traitement (22) sur la compresse principale (4), la membrane (2) présentant sur au moins une partie de la surface en regard de cette compresse une perméabilité permettant des échanges gazeux,
le pansement étant **caractérisé en ce que** l'ouverture d'entrée (34) forme un perçage de la membrane (2), disposé sur le contour adhésif (12) à distance du rebord de cette membrane.

5. Pansement selon la revendication 4, **caractérisé en ce qu'**il comporte en plus de la perméabilité, une ouverture de sortie (40) pour l'air contenu dans la compresse principale (4), l'ouverture de sortie (40) formant un perçage de la membrane (2), disposé sur le contour adhésif (12) à distance du rebord de cette membrane.

6. Pansement selon l'une des revendications 4 et 5, **caractérisé en ce que** le perçage de l'ouverture d'entrée (34) et/ou de sortie (40) comporte en dessous de la membrane (2) un morceau de compresse (30, 38) qui est séparé de la compresse principale (4).

7. Pansement selon l'une quelconque des revendications 4 à 6, **caractérisé en ce qu'**une rondelle rigide (40) est disposée sous la membrane (2) autour du perçage d'entrée (34) pour réaliser un centrage d'un embout (26) de versement du liquide.

8. Pansement selon l'une quelconque des revendications 4 à 6, **caractérisé en ce qu'**il comporte un réservoir intégré prévu pour recevoir le liquide (22), le réservoir intégré communiquant avec le perçage de l'ouverture d'entrée.

9. Pansement selon la revendication 8, **caractérisé en ce que** le réservoir intégré comporte une feuille étanche supérieure (60) fixée par son contour sur la membrane (2), ou sur une feuille étanche inférieure (80) qui est elle-même fixée sur la membrane.

10. Pansement selon l'une des revendications 8 et 9, **caractérisé en ce qu'**il comporte une vanne (66) d'ouverture du réservoir vers la compresse principale (4), commandée par un bouton, le bouton traversant le perçage de l'ouverture d'entrée (34).

11. Pansement selon l'une des revendications 8 à 10, **caractérisé en ce que** la membrane (2) ne comporte pas d'ouverture de sortie en plus de la perméabilité.

12. Pansement selon l'une quelconque des revendications 1 à 7, **caractérisé en ce qu'**il comporte un moyen de fermeture (52) de l'ouverture d'entrée (34), comprenant une partie souple venant sur cette ouverture.

13. Pansement selon l'une quelconque des revendications précédentes, **caractérisé en ce que** la perméabilité permettant des échanges gazeux est formée par une microstructure ou des micro-perforations de la membrane (2).

14. Ensemble comportant un réservoir de liquide et un pansement selon l'une quelconque des revendications 1 à 7, 12 et 13.

## Patentansprüche

1. Hautpflegender Wundverband, umfassend eine Hauptkompresse (4), die mit einer Membran (2) mit einem Haftrand (12) bedeckt ist, der über diese Kompresse hinausragt vorgesehen, um auf die Haut aufgebracht zu werden, und umfassend eine Eintrittsöffnung (6) einer Behandlungsflüssigkeit (22) auf der Hauptkompresse (4), wobei die Membran (2) wenigstens auf einem Teil der Oberfläche dieser Kompresse Gasaustausch erlaubt,
wobei der Wundverband **dadurch gekennzeichnet ist, dass** die Eintrittsöffnung einen Kompressenträger (6) umfasst, der von einer Seite der Hauptkompresse (4) ausgeht, um den Haftrand (12) zu passieren.

2. Wundverband nach Anspruch 1, **dadurch gekennzeichnet, dass** er neben der Durchlässigkeit eine Austrittsöffnung (8) für die in der Hauptkompresse (4) enthaltene Luft umfasst.

3. Wundverband nach Anspruch 2, **dadurch gekennzeichnet, dass** die Austrittsöffnung (8) einen Kompressenträger umfasst, der von einer Seite der Hauptkompresse (4) ausgeht, um den Haftrand (12) zu passieren.

4. Hautpflegender Wundverband, umfassend eine Hauptkompresse (4), die mit einer Membran (2) mit einem Haftrand (12) bedeckt ist, der über diese Kompresse hinausragt, vorgesehen, um auf die Haut aufgebracht zu werden, und der eine Eintrittsöffnung (34) einer Behandlungsflüssigkeit (22) auf der Hauptkompresse (4) umfasst, wobei die Membran (2) wenigstens auf einem Teil der Oberfläche dieser Kompresse Gasaustausch erlaubt, wobei der Wundverband **dadurch gekennzeichnet ist, dass** die Eintrittsöffnung (34) einen Durchstich der Membran (2) bildet, angeordnet auf dem Haftrand (12) entfernt von der Kante dieser Membran.

5. Wundverband nach Anspruch 4, **dadurch gekennzeichnet, dass** er neben der Durchlässigkeit eine Austrittsöffnung (40) für die in der Hauptkompresse (4) enthaltene Luft umfasst, wobei die Austrittsöffnung (40) einen Durchstich der Membran (2) bildet, der auf dem Haftrand (12) entfernt von der Kante dieser Membran angeordnet ist.

6. Wundverband nach einem der Ansprüche 4 und 5, **dadurch gekennzeichnet, dass** der Durchstich der Eintrittsöffnung (34) und/oder der Austrittsöffnung (40) unter der Membran (2) ein Stück Kompresse (30, 38) umfasst, das von der Hauptkompresse (4) getrennt ist.

7. Wundverband nach einem der Ansprüche 4 bis 6, **dadurch gekennzeichnet, dass** eine starre Scheibe (40) unter der Membran (2) um den Eingangsdurchstich (34) angeordnet ist, um eine Zentrierung des Auslaufstutzens (26) der Flüssigkeit herzustellen.

8. Wundverband nach einem der Ansprüche 4 bis 6, **dadurch gekennzeichnet, dass** er einen integrierten Behälter umfasst, vorgesehen, um die Flüssigkeit (22) aufzunehmen, wobei der integrierte Behälter mit dem Durchstich der Eintrittsöffnung in Kommunikation ist.

9. Wundverband nach Anspruch 8, **dadurch gekennzeichnet, dass** der integrierte Behälter eine obere undurchlässige Folie (60) umfasst, befestigt mit ihrer Kontur an der Membran (2) oder an einer unteren undurchlässigen Folie (80), die wiederum selbst an der Membran befestigt ist.

10. Wundverband nach einem der Ansprüche 8 und 9, **dadurch gekennzeichnet, dass** er ein Öffnungsventil (66) des Behälters zur Hauptkompresse (4) hin umfasst, das von einer Taste gesteuert wird, wobei die Taste den Durchstich der Eintrittsöffnung (34) passiert.

11. Wundverband nach einem der Ansprüche 8 bis 10, **dadurch gekennzeichnet, dass** die Membran (2) keine Austrittsöffnung über die Durchlässigkeit hinaus umfasst.

12. Wundverband nach einem der Ansprüche 1 bis 7, **dadurch gekennzeichnet, dass** er ein Verschlussmittel (52) der Eintrittsöffnung (34) umfasst, das einen biegsamen Abschnitt zu dieser Öffnung hin umfasst.

13. Wundverband nach einem der vorhergehenden Ansprüche, **dadurch gekennzeichnet, dass** die Durchlässigkeit die den Gasaustausch erlaubt, durch eine Mikrostruktur oder Mikroperforationen der Membran (2) gebildet ist.

14. Vorrichtung umfassend einen Flüssigkeitsbehälter und einem Wundverband nach einem der Ansprüche 1 bis 7, 12 und 13.

## Claims

1. A dressing for skin care, having a main compress (4) covered by a membrane (2) with an adhesive contour (12) which extends beyond this compress and is designed to be applied to the skin, and having an inlet port (6) for a treatment liquid (22) on the main compress (4), the membrane (2) comprising, on at least part of the surface opposite this compress, a permeability that permits exchange of gases;
**characterised in that** the inlet port comprises a compress arm (6) extending from a side of the main compress (4) to traverse the adhesive contour (12).

2. The dressing according to claim 1, **characterised in that**, in addition to permeability, it has an outlet port (8) for air contained in the main compress (4).

3. The dressing according to claim 2, **characterised in that** the outlet port (8) comprises a compress arm extending from a side of the main compress (4) to traverse the adhesive contour (12).

4. A dressing for skin care, having a main compress (4) covered by a membrane (2) with an adhesive contour (12) which extends beyond this compress and is designed to be applied to the skin, and having an inlet port (34) for a treatment liquid (22) on the main compress (4), the membrane (2) comprising, on at least part of the surface opposite this compress, a permeability that permits exchange of gases;
The dressing being **characterised in that** that the inlet port (34) forms a bore of the membrane (2), provided on the adhesive contour (12) at a distance from the edge of this membrane.

5. The dressing according to claim 4, **characterised in that**, in addition to permeability, it has an outlet port (40) for air contained in the main compress (4), the outlet port (40) forming a bore of the membrane (2), provided on the adhesive contour (12) at a distance from the edge of this membrane.

6. The dressing according to one of the claims 4 and 5, **characterised in that** the bore of the inlet port (34) and/or the outlet port (40) comprises under the membrane (2) a piece of compress (30, 38) which is separated from the main compress (4).

7. The dressing according to any one of the claims 4 to 6, **characterised in that**, a rigid ring (40) is disposed under this membrane (2), around the entry bore (34), to realize a centring of a tip (26) for pouring liquid.

8. Dressing according to any of the claims 4 to 6, **characterised in that** it comprises an integrated reservoir designed to receive the liquid (22), the integrated reservoir communicating with the bore of the inlet port.

9. Dressing according to claim 8, **characterised in that** the integrated reservoir has a tight upper sheet (60) fixed by its contour on the membrane (2) or on a tight lower sheet (80) which itself is fixed on the membrane.

10. Dressing according to one of the claims 8 and 9, **characterised in that** it comprises a valve (66) for opening of the reservoir towards the main compress (4), controlled by a knob, the knob traversing the bore of the inlet port (34).

11. Dressing according to one of the claims 8 to 10, **characterised in that** the membrane (2) does not have an outlet port in addition to permeability.

12. Dressing according to any of the claims 1 to 7, **characterised in that** it comprises a closing member (52) for closing the inlet port (34), comprising a flexible part coming to lie over this port.

13. Dressing according to any of the preceding claims, **characterised in that** the permeability permitting exchange of gases is formed by a microstructure or microperforations of the membrane (2).

14. A set comprising a liquid reservoir and a dressing according to any one of claims 1 to 7, 12 and 13.
